(19) Europäisches Patentamt
European Patent Office
Office européen des brevets

(11) Numéro de publication: **0 547 981 B1**

(12) **FASCICULE DE BREVET EUROPEEN**

(45) Date de publication de fascicule du brevet: **09.08.95**    (51) Int. Cl.⁶: **C07C 253/24**

(21) Numéro de dépôt: **92420446.4**

(22) Date de dépôt: **03.12.92**

(54) **Procédé d'ammoxydation d'hydrocarbures saturés.**

(30) Priorité: **16.12.91 FR 9115843**

(43) Date de publication de la demande:
**23.06.93 Bulletin 93/25**

(45) Mention de la délivrance du brevet:
**09.08.95 Bulletin 95/32**

(84) Etats contractants désignés:
**BE DE ES FR GB IT NL PT**

(56) Documents cités:
**FR-A- 2 072 399**
**FR-A- 2 119 492**
**US-A- 3 433 823**

**PATENT ABSTRACTS OF JAPAN vol. 15, no.
206 (C-835)27 Mai 1991 & JP-A-30 58 961
(NITTO CHEM.IND. CO. LTD.) 14 Mars 1991**

(73) Titulaire: **RHONE-POULENC CHIMIE**
**25, Ouai Paul Doumer**
**F-92408 Courbevoie Cédex (FR)**

(72) Inventeur: **Blanchard, Gilbert**
**5, allée des Acacias,**
**Lagny-le-Sec**
**F-60330 Le Plessis Belleville (FR)**
Inventeur: **Bordes, Elisabeth**
**26, rue Léon Bouchard**
**F-95470 Vemars (FR)**
Inventeur: **Ferre, Gilbert**
**33, avenue Moutiers**
**F-93190 Livry Gargan (FR)**

(74) Mandataire: **Vignally, Noel et al**
**RHONE-POULENC CHIMIE,**
**Direction de la Propriété Industrielle,**
**Centre de Recherches des Carrières,**
**B.P. 62**
**F-69192 Saint-Fons Cédex (FR)**

## Description

La présente invention concerne un procédé d'ammoxydation d'hydrocarbures saturés c'est-à-dire la conversion d'alcanes en un mélange renfermant des nitriles $\alpha,\beta$-insaturés.

Il est bien connu de l'homme de l'art que de très nombreuses propositions ont été formulées en ce qui concerne l'ammoxydation des oléfines et, en particulier celle du propylène. Toutefois, bien que les hydrocarbures saturés, plus largement disponibles, soient des matières premières plus intéressantes au plan économique, il est également bien connu qu'ils ne présentent pas une réactivité comparable dans ce type de réaction pour former notamment des nitriles $\alpha,\beta$-insaturés.

L'une des difficultés rencontrées dans l'ammoxydation des hydrocarbures saturés réside dans la nécessité de pouvoir disposer de catalyseurs susceptibles de déshydrogéner l'hydrocarbure saturé dans des conditions minimisant ou supprimant la combustion de l'ammoniac et/ou celle de l'hydrocarbure tout en assurant une sélectivité raisonnable soit en nitrile $\alpha,\beta$-insaturé (produit visé) par exemple en acrylonitrile au départ du propane, soit en produits valorisables (nitrile précité et oléfine), par exemple, en acrylonitrile et propylène au départ du propane.

Il a déjà été proposé dans le brevet américain n° 3 365 482 d'ammoxyder notamment l'isobutane en méthacrylonitrile sur un catalyseur à base de molybdène déposé sur de l'éta-alumine, dopée par de l'antimoine à 508°C, au départ d'un mélange gazeux renfermant de l'isobutane, de l'air, de l'ammoniac et de la vapeur d'eau (1,0/4,5/1,0/12,5), la sélectivité en méthacrylonitrile atteint 49 % pour un taux de conversion de l'isobutane de 22 %.

Au départ d'un mélange gazeux propane/air/ammoniac/vapeur d'eau (1,0/4,7/0,67/12,8), avec le même catalyseur et à 550°C, la sélectivité en acrylonitrile tombe à 15 % pour un taux de transformation du propane de 29 %.

Dans le brevet français 2 027 238 (correspondant pour partie au brevet américain n° 3 670 009) il est proposé un procédé d'ammoxydation en phase vapeur d'hydrocarbures saturés à une température supérieure à 500°C sur un catalyseur solide qui peut notamment être constitué d'oxyde d'étain, d'oxyde de bore, d'oxyde de molybdène et d'oxyde de silicium. Ainsi, dans l'exemple IX du tableau pages 12-13, la sélectivité en acrylonitrile atteint 35 %, à 32 % de conversion du propane, mais avec des conditions opératoires plaçant le mélange réactionnel propane/ammoniac/air (1/1,2/12) dans le domaine d'explosivité.

Dans le brevet français 2 072 334 (correspondant au brevet britannique n° 1 336 135) il est préconisé un procédé d'ammoxydation catalytique d'alcanes en phase vapeur, à une température inférieure à 500°C avec une forte concentration d'alcane dans le mélange gazeux alimenté, sur un catalyseur solide qui peut notamment être constitué d'oxyde d'étain et d'oxyde de molybdène (90/10 en poids) ; toutefois de meilleures performances sont obtenues avec des catalyseurs constitués d'oxyde d'antimoine et d'oxyde de vanadium.

Dans le brevet français 2 072 399 il est également proposé un procédé d'ammoxydation catalytique d'alcane, en phase vapeur, avec une forte concentration d'alcane dans le mélange gazeux alimenté, sur un catalyseur solide qui peut notamment être un mélange binaire d'oxydes comportant de l'oxyde de molybdène.

Sont plus particulièrement à signaler les couples suivants :

(Mo, Sb) (Mo, Sn) (Mo, V) (Mo, Ti) (Mo, Bi).

Toutefois aucun de ces couples n'offre de performances meilleures que celles obtenues avec des couples ne renfermant pas de molybdène. Les rendements en acrylonitrile obtenus sont très faibles ; au mieux, 1,7 % du propane est converti en acrylonitrile à 570°C sur un catalyseur à base d'oxydes d'étain et de titane.

Dans le brevet français n° 2 119 492 (correspondant au brevet américain n° 3 746 737 et au brevet britannique n° 1 337 759) il est proposé d'utiliser une composition binaire à base d'oxydes de molybdène et de cérium. Toutefois, les performances du couple (Mo, Ce) paraissent insuffisantes en l'absence d'halogène ou de composé halogéné.

Il est en outre préconisé d'ajouter à cette composition binaire (Mo, Ce) un troisième élément choisi parmi le tellure et le bismuth (cf. également US 3 833 638). Là encore, les performances du système catalytique paraissent insuffisantes en l'absence d'halogène ou de composé halogéné. Au surplus, on notera qu'en présence de $CH_3Br$, la sélectivité en acrylonitrile atteint 67 % à 98 % de conversion du propane, mais dans des conditions opératoires plaçant le mélange réactionnel propane/ammoniac/air (1/1, 2/12) dans le domaine d'explosivité.

Dans le brevet français n° 2 119 493 il a également été proposé de réaliser l'ammoxydation d'alcanes en phase vapeur sur un catalyseur solide renfermant des oxydes de bismuth et de molybdène et le cas échéant du phosphore et de la silice.

2

Là encore les performances du système catalytique paraissent insuffisantes en l'absence d'halogène ou de composé halogéné et le mélange réactionnel est dans le domaine d'explosivité.

Face à ces nombreuses insuffisances, divers travaux parallèles ou subséquents ont porté sur l'utilisation de catalyseurs solides à base de vanadium et/ou d'antimoine.

Dans "Chemistry letters 1989 (pp 2173-2176)" des auteurs ont testés dans l'ammoxydation du propane en phase vapeur des oxydes métalliques multicomposants renfermant du molybdène et du bismuth et présentant une structure du type de celle de la scheelite. Il apparaît que, malgré les températures relativement modérées mises en oeuvre, la proportion de produits de combustion (CO, $CO_2$) est très élevée dans tous les cas (au moins 15 %) et que certaines compositions catalytiques testées sont très peu actives vis-à-vis de la réaction souhaitée malgré leur mise en oeuvre dans des conditions se situant dans le domaine d'explosivité ou très proches dudit domaine.

La présence de composé halogéné est susceptible d'induire une corrosion de l'appareillage et n'est donc pas souhaitable dans un procédé industriel.

Il est par ailleurs manifeste que la coproduction de grandes quantités de CO et de $CO_2$ est indésirable à l'échelle industrielle.

Au surplus, l'utilisation de mélanges réactionnels se situant dans le domaine d'explosivité est d'autant moins souhaitable à l'échelle industrielle que l'on met en oeuvre le procédé en lit fixe.

Il apparaît donc qu'il serait hautement souhaitable de disposer d'un procédé d'ammoxydation d'alcanes permettant d'obtenir avec une sélectivité appréciable un mélange de produits valorisables renfermant un nitrile $\alpha,\beta$-insaturé en particulier de l'acrylonitrile, tout en diminuant, pour autant que faire se peut, les pertes de matière première par suite de la formation d'oxydes de carbone.

Il serait également hautement souhaitable de disposer d'un tel procédé dans lequel le catalyseur solide serait relativement simple à préparer et actif en l'absence de promoteur halogéné et pour des mélanges de gaz ne se situant pas nécessairement dans le domaine d'explosivité.

La présente invention a donc pour objet un procédé d'ammoxydation d'alcanes en phase vapeur en présence d'un catalyseur solide dont la phase active renferme du molybdène, du vanadium et de l'oxygène caractérisé en ce que la dite phase active renferme également au moins un élément choisi parmi le manganèse, le zinc, le cobalt, le cuivre, le lithium, le sodium, le potassium et l'argent.

Selon la présente invention des hydrocarbures saturés acycliques ayant de 3 à 12 atomes de carbone par molécule sont mis à réagir en phase vapeur avec de l'ammoniac et de l'oxygène en présence d'un catalyseur dont la phase active vient d'être précisée.

Bien entendu, dans le cadre du présent procédé, on peut utiliser des gaz diluants inertes dans les conditions réactionnelles tels que l'hélium, l'azote et l'argon. De même, de la vapeur d'eau peut être ajoutée au mélange gazeux réactionnel dans de larges limites. Ainsi, le gaz réactif (hydrocarbure saturé, ammoniac, oxygène) peut être dilué par un diluant inerte et/ou par de la vapeur d'eau. Dans cet ensemble la teneur en vapeur d'eau peut varier dans de larges limites en particulier de 0 à 50 % et, de préférence entre 3 et 30 %. Pour une bonne mise en oeuvre du procédé selon l'invention la teneur en gaz réactif sera d'au moins 3 % et de préférence d'au moins 20 %.

Au sein du gaz réactif les teneurs respectives en hydrocarbure saturé, ammoniac et oxygène peuvent varier dans de larges limites.

La teneur en hydrocarbure saturé dans le gaz réactif est de préférence comprise entre 5 et 70 %. Celle en ammoniac est de préférence comprise entre 3 et 45 % et celle en oxygène est de préférence comprise entre 3 et 45 %.

Pour une bonne mise en oeuvre du procédé selon l'invention la composition du mélange réactif sera en dehors du domaine d'explosivité. S'agissant de l'ammoxydation du propane en l'absence de diluant inerte, la composition (propane, oxygène, ammoniac) sera avantageusement choisie à l'intérieur du quadrilatère ABDE figurant au sein du diagramme ternaire ABC représenté sur la figure unique annexée.

Sur ce diagramme ternaire le segment AB représente la teneur en ammoniac de 100 % à 0 % ; le segment BC représente la teneur en propane de 100 à 0 % ; le segment CA représente la teneur en oxygène de 100 à 0 %. Le point D, situé à l'intérieur du segment BC, correspond à une teneur en propane de 45 % ; dans le binaire (propane-$O_2$) ; le point E situé à l'intérieur du segment AC, correspond à une teneur en ammoniac de 79 % dans le binaire ($NH_3$)-$O_2$) %.

Le segment DE délimite le diagramme ternaire en deux parties : un triangle CDE à l'intérieur duquel se situe la zone d'explosivité (déterminée sous 1 bar et à 25°C) et un quadrilatère ABDE à l'intérieur duquel la composition du mélange gazeux réactif sera avantageusement choisie.

S'agissant de l'ammoxydation du propane en présence de gaz diluant(s) inerte(s) et/ou de vapeur d'eau, il conviendra de déterminer la composition du mélange ternaire (propane, oxygène et ammoniac) pour la situer sur le diagramme précité, lorsque le gaz diluant et/ou la vapeur d'eau est en faible proportion.

3

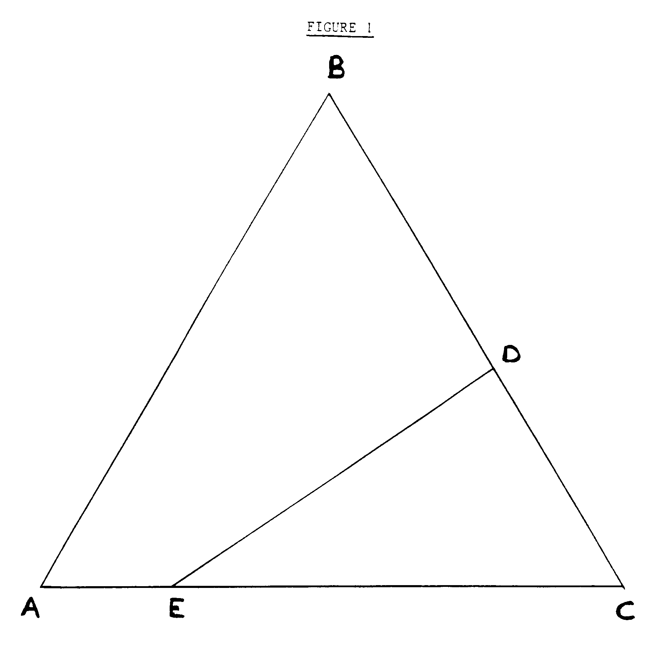

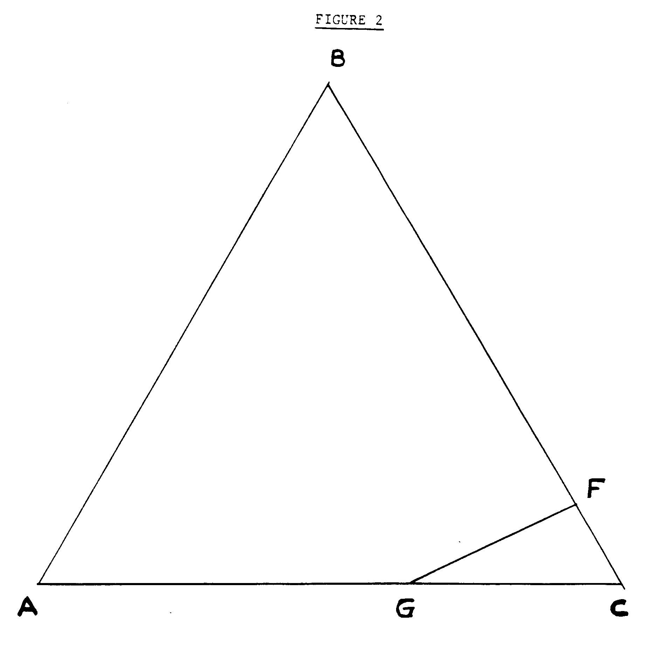

S'agissant de l'ammoxydation du propane au moyen de l'air comme source d'oxygène, la composition (propane, air et ammoniac) sera avantageusement choisie à l'intérieur du quadrilatère ABFG figurant au sein du diagramme ABC représenté sur la figure 2 annexée.

Sur ce second diagramme le segment AB représente la teneur en ammoniac de 100 % à 0 % ; le segment BC représente la teneur en propane de 100 à 0 % : le segment CA représente la teneur en air de 100 à 0 %. Le point F, situé à l'intérieur du segment BC, correspond à une teneur en propane de 16 % dans le binaire (propane-air) ; le point G situé à l'intérieur du segment AC, correspond à une teneur en ammoniac de 35 % dans le binaire (ammoniac-air).

Le segment FG délimite le diagramme ternaire en deux parties : un triangle CFG à l'intérieur duquel se situe la zone d'explosivité (déterminée sous 1 bar à 550°C) et un quadrilatère ABFG à l'intérieur duquel la composition du mélange gazeux réactif sera avantageusement choisie.

Ce second diagramme sera utilisé dans le cas où le mélange oxygène-gaz diluant correspond à une teneur en oxygène équivalente à celle de l'air (≃ 21 % d'oxygène) ou dans le cas où ce mélange est en défaut d'oxygène, par rapport à l'air.

Au départ de propane on obtiendra un mélange renfermant essentiellement du propylène et de l'acrylonitrile. L'acrylonitrile est un intermédiaire industriellement produit sur une large échelle, le propylène est une matière première traditionnellement utilisée pour produire de l'acrylonitrile, et divers autres produits intermédiaires bien connus de l'homme de l'art.

Au départ d'isobutane on obtiendra un mélange renfermant du méthacrylonitrile et de l'iso-butène ou des n-butènes.

Le procédé selon l'invention convient plus particulièrement à l'ammoxydation du propane.

Si l'hydrocarbure saturé mis en oeuvre peut être de qualité technique, il ne renfermera pas de quantités notables de composés à insaturation éthylénique. Ainsi le propane engagé ne renfermera de propylène qu'à l'état de traces.

Le procédé selon l'invention et réalisé sous forme de réaction en phase vapeur. En conséquence n'importe quel dispositif convenant à la réalisation des réactions d'ammoxydation ou d'oxydation en phase vapeur peut être utilisé. Le procédé peut être conduit en continu ou en discontinu et il peut comprendre l'utilisation d'un lit fixe ou d'un lit fluidisé.

La température de réaction est en général comprise entre 350 et 550°C et, de préférence entre 410 et 510°C.

La pression totale du mélange réactionnel peut être supérieure ou égale à la pression atmosphérique. Elle est généralement comprise entre 1 et 6 bar et, de préférence entre 1 et 4 bar.

Le débit gazeux est fixé de telle sorte que la vitesse volumique horaire soit comprise entre 100 et 36000 $h^{-1}$ et, de préférence entre 200 et 20000 $h^{-1}$.

Bien entendu, l'homme de l'art sera à même de trouver un compromis entre la température, le débit gazeux, la nature précise du catalyseur mise en oeuvre et les divers autres paramètres de la réaction compte-tenu de ses objectifs de production.

Dans le procédé selon la présente invention on met en oeuvre un catalyseur solide dont la phase active répond à la formule empirique

$$Mo_a \, V_b \, M_c \, O_x$$

dans laquelle :
- M représente un ou plusieurs éléments choisis parmi le manganèse, le zinc, le cobalt, le cuivre, le lithium, le sodium, le potassium et l'argent.
- a est un nombre non nul et inférieur à 2 ;
- b est un nombre non nul et inférieur à 2 ;
- c est un nombre non nul et inférieur à 1 ; et
- x est déterminé par le degré d'oxydation des autres éléments.

De préférence, M représente un ou plusieurs éléments dont au moins un est choisi parmi le manganèse, le zinc et le cobalt.

De préférence, dans la formule empirique en cause
- a est compris entre 0,1 et 1 inclus ;
- b est compris entre 0,05 et 1,8 inclus ; et
- c est compris entre 0,1 et 1 inclus.

Sont plus particulièrement préférées des phases actives répondant à la formule empirique en cause et dans laquelle :
- M représente un élément choisi parmi le manganèse, le zinc et le cobalt

EP 0 547 981 B1

- a est compris entre 0,1 et 1 inclus ;
- b est compris entre 0,05 et 1,8 inclus ;
- c est compris entre 0,1 et 1 inclus ; et

Les phases actives en cause peuvent être mises en oeuvre dans le cadre du procédé selon l'invention sous forme massique ou à l'état particulaire. Ces phases peuvent être utilisées sous forme de poudres ou de billes, extrudées ou concassées, par exemple.

Elles peuvent être également déposées sur support inerte ou l'enrober. La nature du support n'est pas critique dès lors qu'il est chimiquement inerte vis-à-vis des réactifs dans les conditions réactionnelles choisies. A titre d'exemples de supports susceptibles de convenir à la préparation de catalyseurs utilisables dans le cadre du procédé selon l'invention, on peut citer : la silice, l'alumine, la silice-alumine, l'argile frittée, le carborandum, la magnésie, le silicate de magnésium, la terre de diatomée. Ce support est de préférence non poreux et peut être notamment, à base d'oxyde réfractaire sous forme particulaire, le support le plus couramment employé étant à base d'argile. Ce support peut par exemple être constitué de billes d'argile, inertes, pleines, solides et rugueuses, de diamètre compris entre 0,5 et 6 mm. La valeur précise du diamètre des billes pourra être choisie par l'homme de l'art en fonction de la perte de charge admissible dans le réacteur. Le support peut également être rendu non poreux par émaillage.

Le support peut également être un substrat céramique, ledit substrat étant de préférence sous forme d'une structure inerte et rigide de type monolithique comprenant des canaux ou conduits. De tels supports sont bien connus de l'homme de l'art et ont été largement décrits dans la littérature. Les substrats en matière céramique utilisés sont notamment ceux comportant comme matière principale la cordiérite, l'alumine, la mullite, la porcelaine, les carbures de bore ou de silicium.

Lorsqu'on fait appel à un catalyseur enrobé, la quantité de phase active qui peut varier dans de larges limites, est en pratique comprise entre 5 et 35 % et, de préférence entre 8 et 20 % en poids par rapport à l'ensemble (support + phase active).

La préparation des catalyseurs mis en oeuvre dans le procédé selon l'invention peut être réalisée de diverses manières connues en soi, tel le mélange de sels convenables des constituants élémentaires dans l'eau ou dans un autre solvant suivi de l'évaporation jusqu'à siccité, ou de la précipitation par ajout d'une base telle l'ammoniaque ou d'un acide tel l'acide chlorhydrique, ou l'atomisation d'une suspension obtenue après mélange des sels convenables.

Les sels convenables les plus couramment employés sont solubles dans l'eau et contiennent des anions et des cations qui peuvent être décomposés par la chaleur lors des étapes ultérieures. Ce sont, par exemple, l'heptamolybdate d'ammonium pour le molybdène, le vanadate d'ammonium pour le vanadium, les nitrates ou chlorures de manganèse, de zinc et de cobalt.

Une fois le mélange des sels réalisé, un précurseur peut être obtenu par la méthode dite d'évaporation. L'eau de la suspension obtenue est évaporée en chauffant entre 20 et 100°C sous agitation pendant le temps nécessaire à l'obtention d'une pâte non coulante. L'agitation et le chauffage sont alors arrêtés.

La pâte ainsi obtenue, étalée sur une épaisseur de 2 cm environ, est séchée sous air à 120°C environ pendant 15 h environ. Le précurseur ainsi obtenu peut ensuite être broyé et calciné entre 200 et 1000°C, de préférence ente 400 et 600°C, pendant au moins 30 mn, de préférence au moins une heure : il peut s'avérer utile de faire successivement plusieurs opérations de broyage et calcination. La calcination peut s'effectuer en montant progressivement la température, 100 à 200°C par heure par exemple, en raison, notamment, des risques liés à la décomposition exothermique du nitrate d'ammonium vers 230°C. La phase active ainsi obtenue après refroidissement peut ensuite être broyée pour que sa granulométrie n'excède pas 400 μm environ.

Le précurseur peut aussi être obtenu selon une variante comprenant la précipitation avec addition, par exemple, d'ammoniaque ou d'acide chlorhydrique en fin de mélange des sels pour stabiliser le pH vers 7 environ. Il est préférable de chauffer la suspension entre 20 et 100°C pendant environ une heure pour parfaire la précipitation des espèces.

Puis la suspension est filtrée et lavée. Le gâteau de filtration est ensuite étalé, puis séché, broyé et calciné selon les conditions décrites ci-avant dans le cadre de la méthode d'évaporation, pour donner la phase active. Plusieurs phases actives peuvent être mélangées pour donner une nouvelle phase active, par exemple dans un mortier.

Certains catalyseurs, utiles pour une mise en oeuvre du procédé en lit fixe, peuvent être obtenus par enrobage de manière connue en soi, des phases actives, intermédiaires ou finies, broyées. Cette méthode classique consiste à déposer autour de billes inertes mais rugueuses une couche de phase active intermédiaire ou finie.

Une fois les billes recouvertes de la quantité voulue de la phase active, elles sont séOhées par de l'air chaud, entre 70 et 150°C pendant au moins 30 minutes, puis introduites dans un four pour être calcinées

5

entre 300 et 600°C de préférence entre 450 et 500°C, pendant au moins 3 heures.

Certains catalyseurs utiles pour une mise en oeuvre du procédé selon l'invention en lit mobile ou lit fluidisé peuvent être obtenus par la technique en soi connue du séchage par atomisation en atmosphère, de préférence, non réductrice. Par une telle opération, le cas échéant suivie d'une calcination à une température de l'ordre de 400 à 1100°C, on obtient des poudres de forme sphérique de diamètre compris entre 5 et 700 $\mu$m. Des poudres constituées pour au moins 80 % en poids de particules dont la dimension est comprise entre 5 et 100 $\mu$m sont préférées dans la cadre d'une utilisation en lit fluidisé.

Les produits de la réaction peuvent être récupérés dans les gaz effluents par tout moyen approprié. Par exemple les gaz effluents peuvent passer dans un condenseur contenant de l'acide sulfurique dilué pour neutraliser l'ammoniac non converti. Les gaz peuvent ensuite passer sur une colonne absorbante réfrigérée pour condenser l'acrylonitrile, l'acétonitrile et l'acide cyanhydrique, les vapeurs non condensées contenant principalement du propane non converti du propylène, des hydrocarbures légers et le cas échéant du $CO_2$. On peut ensuite séparer l'acrylonitrile et l'acide cyanhydrique de l'acétonitrile par distillation, puis distiller à son tour le mélange acrylonitrile-acide cyanhydrique récupéré pour séparer l'acrylonitrile de l'acide cyanhydrique.

Les exemples ci-après illustrent la présente invention.

EXEMPLE 1 : Préparation d'un catalyseur (A) dont la phase active répond à la formule $Mn_{0,8}V_{1,6}Mo_{0,4}O_6$

On prépare une phase active de composition $Mn_{0,8}V_{1,6}Mo_{0,4}O_6$ selon le protocole opératoire suivant : on prépare une solution (a) de nitrate de manganèse par dissolution de 204,91 g de $Mn(NO_3)_2$, $4H_2O$ 98 % (commercialisé par la Société PROLABO) dans 300 cm3 d'eau permutée, une solution (b) d'heptamolybdate d'ammonium par dissolution de 70,64 g de $(NH_4)_6Mo_7O_{24}$,$4H_2O$ (commercialisé par la Société MERCK) dans 200 cm3 d'eau permutée, et une suspension dans 400 cm3 d'eau permutée de 187,17 g de $NH_4VO_3$ - (commercialisé par la Société PROLABO). On verse la solution (a) dans la suspension (c) dans un réacteur agité. On introduit ensuite, toujours sous agitation, la solution (b). On agite fortement et monte la température progressivement jusqu'à 100-110°C. La pâte obtenue est séchée à 120°C pendant 15 h environ. Le produit obtenu est ensuite broyé, calciné à 600°C pendant 6 h, rebroyé et recalciné à 600°C pendant 42 h.

Le produit (I) ainsi obtenu présente une surface spécifique mesurée selon la méthode B.E.T. de à 0,1 $m^2g^{-1}$.

20 g du produit (I) sont saupoudrés lentement sur 123 g de support inerte composé de billes d'argile de diamètre moyen 4,8 mm, préalablement placées dans un drageoir en rotation et humidifiées par du glucose en solution aqueuse à 10 %. Dès que les billes sont sèches à l'extérieur, nous pulvérisons une petite quantité de la solution de glucose. Puis nous saupoudrons à nouveau le produit (I) sur les billes. Nous poursuivons ces opérations alternativement jusqu'à enrobage de la totalité du produit (I). On sèche ensuite à 120°C pendant 2 h et calcine à 480°C pendant 6 h.

Le catalyseur (A) ainsi obtenu, conforme à l'invention, est constitué de 11,6 % en poids de $Mn_{0,8}V_{1,6}Mo_{0,4}O_6$ déposés sur billes d'argile.

EXEMPLE 2 - Préparation d'un catalyseur (B) dont la phase active répond à la formule $Mn_{0,8}V_{1,6}Mo_{0,4}O_6$

On prépare une phase active de composition $Mn_{0,8}V_{1,6}Mo_{0,4}O_6$ selon le même protocole opératoire que dans l'exemple 1, excepté les calcinations finales que l'on effectue de la façon suivante : le produit est broyé, calciné à 450°C pendant 8 h, rebroyé, recalciné à 450°C pendant 8 h, rebroyé, calciné à 550°C pendant 4 h, rebroyé et recalciné à 550°C pendant 4 h.

Le produit (I) ainsi obtenu et présente une surface spécifique mesurée selon la méthode B.E.T. de 1,7 $m^2g^{-1}$.

20 g du produit (I) sont saupoudrés lentement sur 123 g de support inerte composé de billes d'argile de diamètre moyen 4,8 mm, préalablement placées dans un drageoir en rotation et humidifiées par du glucose en solution aqueuse à 10 %. Dès que les billes sont sèches à l'extérieur, nous pulvérisons une petite quantité de la solution de glucose. Puis nous saupoudrons à nouveau le produit (I) sur les billes. Nous poursuivons ces opérations alternativement jusqu'à enrobage de la totalité du produit (I). On sèche ensuite à 120°C pendant 2 h et calcine à 480°C pendant 6 h.

Le catalyseur (B) ainsi obtenu, conforme à l'invention, est constitué de 10 % en poids de $Mn_{0,8}V_{1,6}Mo_{0,4}O_6$ déposés sur billes d'argile.

EXEMPLE 3 - Préparation d'un catalyseur (C) dont la phase active répond à la formule $Zn_{0,85}V_{1,7}Mo_{0,3}O_6$

On prépare une phase active de composition $Zn_{0,85}V_{1,7}Mo_{0,3}O_6$ selon le protocole opératoire suivant : on prépare une solution (a) de nitrate de zinc par dissolution de 252,8 g de $Zn(NO_3)_2$, $6H_2O$ (commercialisé par la Société PROLABO) dans 250 cm3 d'eau permutée, une solution (b) d'heptamolybdate d'ammonium par dissolution de 52,95 g de $(NH_4)_6Mo_7O_{24}$,$4H_2O$ (commercialisé par la Société MERCK) dans 150 cm3 d'eau permutée, et une suspension dans 210 cm3 d'eau permutée de 165.75 g de $NH_4VO_3$ (commercialisé par la Société PROLABO). On mélange les deux solutions (a) et (b) et on verse ce mélange dans la suspension (c) dans un réacteur agité. On laisse sous agitation pendant 20 h et monte la température progressivement jusqu'à 100-110°C. On laisse à reflux pendant 1 h et évapore. La pâte obtenue est séchée à 120°C pendant 15 h environ. Le produit obtenu est ensuite broyé, calciné à 250°C pendant 4 h, rebroyé et calciné à 450°C pendant 8 h, rebroyé et recalciné à 450°C pendant 8 h, rebroyé et calciné à 550°C pendant 4 h, rebroyé et recalciné à 550°C pendant 4 h.
Le produit (I) ainsi obtenu présente une surface spécifique mesurée selon la méthode B.E.T. de 0,8 $m^2g^{-1}$.

20 g du produit (I) sont saupoudrés lentement sur 123 g de support inerte composé de billes d'argile de diamètre moyen 4,8 mm, préalablement placées dans un drageoir en rotation et humidifiées par du glucose en solution aqueuse à 10 %. Dès que les billes sont sèches à l'extérieur, nous pulvérisons une petite quantité de la solution de glucose. Puis nous saupoudrons à nouveau le produit (I) sur les billes. Nous poursuivons ces opérations alternativement jusqu'à enrobage de la totalité du produit (I). On sèche ensuite à 120°C pendant 2 h et calcine à 480°C pendant 6 h.
Le catalyseur (C) ainsi obtenu, conforme à l'invention, est constitué de 12 % en poids de $Zn_{0,85}V_{1,7}Mo_{0,3}O_6$ déposés sur billes d'argile.

EXEMPLE 4 - Préparation d'un catalyseur (D) dont la phase active répond à la formule $Co_{0,9}V_{1,8}Mo_{0,2}O_6$

On prépare une phase active de composition $Co_{0,9}V_{1,8}Mo_{0,2}O_6$ de la façon suivante : on met en suspension dans 100 cm3 d'eau permutée 84 g de $NH_4VO_3$, on prépare une solution d'heptamolybdate d'ammonium par dissolution de 14 g de $(NH_4)_6Mo_7O_{24}$,$4H_2O$ dans 100 cm3 d'eau permutée et une solution de nitrate de cobalt par dissolution de 105 g de $Co(NO_3)_2$,$6H_2O$ dans 100 cm3 d'eau permutée. On coule la solution d'heptamolybdate d'ammonium sur la suspension de métavanadate d'ammonium, dans un réacteur agité. On introduit ensuite, toujours sous agitation, la solution de nitrate de cobalt. Après 20 h d'agitation à température ambiante, on évapore à sec, sèche à 120°C, puis calcine à 250°C pendant 4 h. Le produit obtenu est broyé, et est soumis au cycle calcinations-broyages suivant :
450°C pendant 4 h - broyage
450°C pendant 4 h - broyage
450°C pendant 8 h - broyage
550°C pendant 4 h - broyage
550°C pendant 4 h - broyage
Le produit (I) ainsi préparé présente une surface spécifique mesurée selon la méthode B.E.T. de 2 $m2g^{-1}$.

15 g du produit (I) sont saupoudrés lentement sur 100 g de support inerte composé de billes d'argile de diamètre moyen 4,8 mm, préalablement placées dans un drageoir en rotation et humidifiées par du glucose en solution aqueuse à 10 %. Dès que les billes sont sèches à l'extérieur, nous pulvérisons une petite quantité de la solution de glucose. Puis nous saupoudrons à nouveau le produit (I) sur les billes. Nous poursuivons ces opérations alternativement jusqu'à enrobage de la totalité du produit (I). On sèche ensuite à 120°C pendant 2 h et calcine à 480°C pendant 6 h.
Le catalyseur (D) ainsi obtenu, conforme à l'invention, est constitué de 12 % en poids de $Co_{0,9}V_{1,8}Mo_{0,2}O_6$ déposés sur billes d'argile.

EXEMPLE 5 - Préparation d'un catalyseur (E) dont la phase active répond à la formule $Mn_{0,8}V_{1,6}Mo_{0,4}O_6$

On prépare un produit (I), de composition $Mn_{0,6}V_{1,2}Mo_{0,8}O_6$, selon le protocole opératoire suivant : on prépare une solution (a) de nitrate de manganèse par dissolution de 153,7 g de $Mn(NO_3)_2$, $4H_2O$ dans 225 cm3 d'eau permutée, une solution (b) d'heptamolybdate d'ammonium par dissolution de 141,28 g de $(NH_4)_6Mo_7O_{24}$,$4H_2O$ dans 400 cm3 d'eau permutée, et une suspension (c) de 140 g de $NH_4VO_3$ dans 150 cm3 d'eau permutée. On verse le mélange des solutions (a) et (b) dans la suspension (c) dans un réacteur agité. On agite pendant une vingtaine d'heures et monte la température progressivement jusqu'à 100-110°C. La pâte obtenue est séchée à 120°C pendant 15 h environ. Le produit est calciné sous air à 250°C pendant 4 h, broyé, calciné à 450°C pendant 8 h, rebroyé, recalciné à 450°C pendant 8 h, rebroyé, calciné à 550°C pendant 4 h, rebroyé et recalciné à 550°C pendant 4 h.

On prépare ensuite un produit (II), de composition $\alpha MnV_2O_6$, selon le protocole opératoire décrit par R. KOZLOWSKI, J. ZIOLKOWSKI, K. MOCALA & J. HABER, J. Sol. State Chem. 35, 1-9 (1980) : on prépare une solution de nitrate de manganèse par dissolution de 256,13 g de $Mn(NO_3)_2,4H_2O$ dans 300 cm3 d'eau permutée. On met en suspension dans 200 cm3 d'eau permutée 116,98 g de $NH_4VO_3$. On verse la solution de nitrate de manganèse dans la suspension de métavanadate d'ammonium, dans un réacteur agité. Après 10 à 15 mn d'agitation à température ambiante, on filtre sur verre fritté et lave par 2 litres d'eau permutée. Le produit obtenu est ensuite séché à 120°C pendant 15 h environ puis calciné sous air pendant 5 h à 500°C.

On mélange dans un mortier 15 g du produit (I) et 15 g du produit (II) ainsi obtenus.

Le produit (III) ainsi préparé, de composition globale $Mn_{0,8}V_{1,6}Mo_{0,4}O_6$, présente une surface spécifique mesurée selon la méthode B.E.T. de 3 $m^2g^{-1}$.

10 g du produit (III) sont saupoudrés lentement sur 67 g de support inerte composé de billes d'argile de diamètre moyen 4,8 mm, préalablement placées dans un drageoir en rotation et humidifiées par du glucose en solution aqueuse à 10 %. Dès que les billes sont sèches à l'extérieur, nous pulvérisons une petite quantité de la solution de glucose. Puis nous saupoudrons une petite quantité de la solution de glucose. Puis nous saupoudrons à nouveau le produit (III) sur les billes. Nous poursuivons ces opérations alternativement jusqu'à enrobage de la totalité du produit (III). On sèche ensuite à 120°C pendant 2 h et calcine à 480°C pendant 6 h.

Le catalyseur (E) ainsi obtenu, conforme à l'invention, est constitué de 12,4 % en poids de $Mn_{0,8}V_{1,6}Mo_{0,4}O_6$ déposés sur billes d'argile.

EXEMPLE 6 : Préparation d'un catalyseur (F) dont la phase active répond à la formule $Mn_{0,4}V_{0,05}Mo_{0,4}0_{1,75}$

On prépare un produit (I) de composition $Mn_{0,4}V_{0,05}Mo_{0,4}0_{1,75}$ selon le protocole opératoire suivant : on prépare une solution (a) de chlorure de manganèse par dissolution de 79,16 g de $MnCl_2, 4H_2O$ dans 300 cm3 d'eau permutée, et une solution (b) d'heptamolybdate d'ammonium par dissolution de 70,6 g de $(NH_4)_6Mo_7O_{24},4H_2O$ dans 300 cm3 d'eau permutée. On ajoute 4,52 g de $V_2O_5$ puis la solution (b) à la solution (a) dans un réacteur agité. On ajoute ensuite 40 cm3 de $NH_4OH$ (20 % $NH_3$) et chauffe à reflux pendant 3 h. On filtre sur verre fritté, lave par 1 l d'eau permutée. Le produit obtenu est ensuite séché à 120°C pendant 15 h environ, puis calciné sous air à 500°C pendant 4 h.

10 g du produit (I) ainsi obtenu sont saupoudrés lentement sur 67 g de support inerte composé de billes d'argile de diamètre moyen 4,8 mm, préalablement placées dans un drageoir en rotation et humidifiées par du glucose en solution aqueuse à 10 %. Dès que les billes sont sèches à l'extérieur, nous pulvérisons une petite quantité de la solution de glucose. Puis nous saupoudrons à nouveau le produit (I) sur les billes. Nous poursuivons ces opérations alternativement jusqu'à enrobage de la totalité du produit (I). On sèche ensuite à 120°C pendant 2 h et calcine à 480°C pendant 6 h.

Le catalyseur (F) ainsi obtenu, conforme à l'invention, est constitué de 9,1 % en poids de $Mn_{0,4}V_{0,05}Mo_{0,4}0_{1,75}$ déposés sur billes d'argile.

Essai témoin (a) : Préparation d'un catalyseur (a) qui n'entre pas dans le cadre de l'invention et dont la phase active répond à la formule $MnV_2O_6$.

Le produit (II) préparé à l'exemple 5 ci-avant présente une surface spécifique mesurée selon la méthode B.E.T. de 5 $m^2g^{-1}$. Ce produit est appelé produit (I) dans le présent exemple.

20 g du produit (I) sont saupoudrés lentement sur 123 g de support inerte composé de billes d'argile de diamètre moyen 4,8 mm, préalablement placées dans un drageoir en rotation et humidifiées par du glucose en solution aqueuse à 10 %. Dès que les billes sont sèches à l'extérieur, nous pulvérisons une petite quantité de la solution de glucose. Puis nous saupoudrons à nouveau le produit (I) sur les billes. Nous poursuivons ces opérations alternativement jusqu'à enrobage de la totalité du produit (I). On sèche ensuite à 120°C pendant 2 h et calcine à 480°C pendant 6 h.

Le catalyseur (a) ainsi obtenu, non conforme à l'invention, est constitué de 13 % en poids de $\alpha MnV_2O_6$ déposés sur billes d'argile.

Essai témoin (b) : Préparation d'un catalyseur (b) qui n'entre pas dans le cadre de l'invention et dont la phase active répond à la formule $ZnV_2O_6$.

On prépare une solution de nitrate de Zinc par dissolution de 148,7 g de $Zn(NO_3)_2,6H_2O$ (commercialisé par la Société PROLABO) dans 200 m3 d'eau permutée. On met en suspension dans 200 cm3 d'eau permutée 117 g de $NH_4VO_3$ (commercialisé par la Société PROLABO). On verse la solution de nitrate de zinc dans la suspension de métavanadate d'ammonium, dans un réacteur agité. Après 23 h d'agitation à température ambiante, on filtre sur verre fritté et lave par 400 cm3 d'eau permutée. Le produit obtenu est ensuite séché à 120°C pendant 15 h environ puis calciné sous air pendant 5 h à 500°C.

Le produit (I) ainsi obtenu présente une surface spécifique mesurée selon la méthode B.E.T. de 3 $m^2g^{-1}$.

20 g du produit (I) sont saupoudrés lentement sur 123 g de support inerte composé de billes d'argile de diamètre moyen 4,8 mm, préalablement placées dans un drageoir en rotation et humidifiées par du glucose en solution aqueuse à 10 %. Dès que les billes sont sèches à l'extérieur, nous pulvérisons une petite quantité de la solution de glucose. Puis nous saupoudrons à nouveau le produit (I) sur les billes. Nous poursuivons ces opérations alternativement jusqu'à enrobage de la totalité du produit (I). On sèche ensuite à 120°C pendant 2 h et calcine à 480°C pendant 6 h.

Le catalyseur (b) ainsi obtenu, non conforme à l'invention, est constitué de 16 % en poids de $ZnV_2O_6$ déposés sur billes d'argile.

Essai témoin (c) : Préparation d'un catalyseur (c) qui n'entre pas dans le cadre de l'invention et dont la phase active répond à la formule $CoV_2O_6$

On prépare une phase active de composition $CoV_2O_6$ de la façon suivante : on met en suspension dans 200 cm3 d'eau permutée 117 g de $NH_4VO_3$ et on prépare une solution de nitrate de cobalt par dissolution de 145,5 g de $Co(NO_3)_2,6H_2O$ dans 200 cm3 d'eau permutée. On coule la solution de nitrate de cobalt sur la suspension de métavanadate d'ammonium, dans un réacteur agité. Après 23 h d'agitation à température ambiante, on filtre sur verre fritté et lave par 3 fois 200 cm3 d'eau permutée. Le produit obtenu est ensuite séché à 120°C pendant 15 h environ puis calciné sous air pendant 5 h à 500°C.

Le produit (I) ainsi préparé présente une surface spécifique mesurée selon la méthode B.E.T. de 3,6 $m^2g^{-1}$.

15 g du produit (I) sont saupoudrés lentement sur 100 g de support inerte composé de billes d'argile de diamètre moyen 4,8 mm, préalablement placées dans un drageoir en rotation et humidifiées par du glucose en solution aqueuse à 10 %. Dès que les billes sont sèches à l'extérieur, nous pulvérisons une petite quantité de la solution de glucose. Puis nous saupoudrons à nouveau le produit (I) sur les billes. Nous poursuivons ces opérations alternativement jusqu'à enrobage de la totalité du produit (I). On sèche ensuite à 120°C pendant 2 h et calcine à 480°C pendant 6 h.

Le catalyseur (c) ainsi obtenu, non conforme à l'invention, est constitué de 12 % en poids de $CoV_2O_6$ déposés sur billes d'argile.

MODE OPERATOIRE GENERAL DES TESTS D'AMMOXYDATION -

L'échantillon de catalyseur est préalablement porté sur un banc de mesure à la température de 150°C sous balayage d'hélium pendant 10 mn, puis il est soumis à un flux gazeux dont la composition sera précisée pour chaque exemple et qui renferme du propane, de l'ammoniac, de l'oxygène, de la vapeur d'eau et de l'hélium.

La pression totale du mélange réactionnel est de 1,3 bar abs.

Le débit gazeux est défini de façon à avoir une vitesse volumique horaire (VVH) de 1000 $h^{-1}$, sauf mention contraire.

Le principe du test d'ammoxydation du propane est le suivant :
- On porte le catalyseur à une température $T_1$, par exemple 300°C, et après 30 mn de stabilisation à la température $T_1$ on détermine par chromatographie en phase gazeuse la composition du mélange en sortie de réacteur.
- On calcule les pourcentages de conversion et les sélectivités obtenus sur le catalyseur examiné à la température d'entrée $T_1$ par des relations du type :

Conversion du propane = % propane transformé / % propane introduit Sélectivité en acrylonitrile = % propane transformé en acrylonitrile / % propane converti

- On porte ensuite le catalyseur de 300 à 550°C par incrément de 20 à 30°C et on détermine toutes les 40 mn les pourcentages de conversion et les sélectivités.

Dans les exemples ci-après, les conventions suivantes sont utilisées :

TTC3H8 = conversion du propane
SACN = sélectivité en acrylonitrile
SACN + C3H6 = sélectivité en acrylonitrile et propylène
SCOX = sélectivité en monoxyde et dioxyde de carbone.

EXEMPLE 7 ; essai témoin (d) -

On détermine les performances des catalyseurs (A) et (a) à diverses températures et dans les conditions communes suivantes :
. volume de catalyseur (phase active + billes d'argile) = 20 cm3
. Débit total du mélange synthétique = 20 l.h$^{-1}$
. Composition dudit mélange =
  $C_3H_8$ = 19,0 % (% en volume)
  $NH_3$ = 7,5 %
  $O_2$ = 10,0 %
  $H_2O$ = 25,0 %
  He = 38,5 %

Les résultats et les conditions particulières figurent au tableau I ci-après :

TABLEAU I

| REF. | CATALYSEUR | T°C | TTC3H8 (%) | SACN (%) | SACN + C3H6(%) | SCOX (%) |
|---|---|---|---|---|---|---|
| témoin d | (a) | 420 | 11 | 12 | 34 | 22 |
| | | 445 | 12 | 2,5 | 35 | 27 |
| | | 470 | 15 | 0 | 37 | 31 |
| Exem. 7 | (A) | 420 | 2 | 8 | 79 | 0 |
| | | 470 | 11 | 25 | 63 | 2 |
| | | 510 | 19 | 29 | 58 | 11 |

EXEMPLE 8 ; essai témoin (e) :

On détermine les performances des catalyseurs (C) et (b) à 480°C et dans les conditions communes suivantes :
. volume de catalyseur (phase active + billes d'argile) = 25 cm3
. Débit total du mélange synthétique = 25 l.h$^{-1}$
. Composition dudit mélange =
  $C_3H_8$ = 20,0 % (% en volume)
  $NH_3$ = 5,0 %
  $O_2$ = 15,0 %
  $H_2O$ = 20,0 %
  He = 40,0 %

Les résultats et les conditions particulières figurent au tableau II ci-après :

TABLEAU II

| REF. | CATALYSEUR | TTC3H8 (%) | SACN (%) | SACN + C3H6(%) | SCOX (%) |
|---|---|---|---|---|---|
| témoin e | (b) | 14 | 0 | 44 | 39 |
| exemple 8 | (C) | 9 | 10 | 38 | 17 |

EXEMPLE 9 : Essai témoin (f) :

On détermine les performance des catalyseurs (D) et (c) à diverses températures et dans les conditions communes suivantes :

. volume de catalyseur (phase active + billes d'argile) = 25 cm3
. Débit total du mélange synthétique = 25 l.h$^{-1}$
. Composition dudit mélange =
   $C_3H_8$ = 7,5 % (% en volume)
   $NH_3$ = 15,0 %
   $O_2$ = 15,0 %
   $H_2O$ = 20,0 %
   He = 42,5 %

Les résultats et les conditions particulières figurent au tableau III ci-après :

TABLEAU III

| REF. | CATALYSEUR | T°C | TTC3H8 (%) | SACN (%) | SACN + C3H6(%) | SCOX (%) |
|------|-----------|-----|-----------|----------|----------------|----------|
| témoin f | (c) | 415 | 4 | 1 | 89 | 1 |
|          |     | 435 | 9 | 2 | 57 | 25 |
|          |     | 455 | 19 | 0 | 26 | 31 |
| exemple 9 | (D) | 415 | 5 | 12 | 78 | 0 |
|           |     | 435 | 8 | 8 | 72 | 9 |
|           |     | 455 | 16 | 18 | 51 | 16 |

EXEMPLES 10 A 21 -

On détermine les performances de divers catalyseurs dont la préparation est l'objet des exemples 1 à 6 ci-avant, dans diverses conditions sous une vitesse spatiale horaire (VVH) de 1000 h$^{-1}$.

La référence du catalyseur utilisé, les conditions particulières mises en oeuvre ainsi que les résultats obtenus figurent dans le tableau IV ci-après :

TABLEAU IV

| Référence | Exemple 10 | Exemple 11 | Exemple 12 | Exemple 13 | Exemple 14 | Exemple 15 | Exemple 16 | Exemple 17 |
|---|---|---|---|---|---|---|---|---|
| CATALYSEUR | (A) | (B) | (A) | (B) | (D) | (E) | (E) | (F) |
| Composition(*) | % Volume | % Volume | % Volume | % Volume | % Volume | % Volume | % Volume | % Volume |
| $C_3H_8$ | 11 / 19 / 26 | 20 / 25 | 19 / 16,5 | 20 / 25 | 40 | 48 | | 40 |
| $NH_3$ | 7,5 | 15 | 7,5 | 15 | 15 | 9 | | 15 |
| $O_2$ | 10 | 15 / 5 | 10 / 4 | 15 / 5 | 15 | 18 | | 15 |
| $H_2O$ | 25 | 20 | 25 | 20 | 20 | 20 | | 20 |
| He | qsp 100 % | qsp 100 % | qsp 100 % | qsp 100 % | 10 | 5 | | 10 |
| T°C | 470 | 460 | 470 | 460 | 440 / 460 / 480 | 430 | 450 / 470 | 480 |
| % TT $C_3H_8$ | 9 / 11 / 3 | 11 / 11 | 6 / 11 / 12 | 5 / 11 / 7 | 15 / 14 / 14 | 8 | 8 / 9 | 10 |
| S AN | 18 / 25 / 7 | 20 / 14 | 16 / 25 / 21 | 12 / 14 / 7 | 22 / 22 / 27 | 14 | 12 / 12 | 18 |
| S AN+$C_3H_6$ | 65 / 63 / 88 | 59 / 48 | 78 / 63 / 54 | 81 / 48 / 68 | 57 / 60 / 64 | 63 | 62 / 64 | 69 |
| S COX | 8 / 2 / 0 | 0 / 0 | 5 / 2 / 12 | 0 / 0 / 0 | 0 / 0,2 / 0 | 6 | 5 / 9 | 4 |

| Référence | Exemple 18 | Exemple 19 | Exemple 20 | Exemple 21 |
|---|---|---|---|---|
| CATALYSEUR | (D) | (D) | (F) | (F) |
| Composition(*) | % Volume | % Volume | % Volume | % Volume |
| $C_3H_8$ | 25 | 25 | 25 | 7,5 |
| $NH_3$ | 10 | 25 | 10 | 15 |
| $O_2$ | 25 | 10 | 25 | 15 |
| $H_2O$ | 20 | 20 | 20 | 20 |
| He | 20 | 20 | 20 | 42,5 |
| T°C | 420 | 440 / 460 | 440 / 460 | 460 / 480 |
| % TT $C_3H_8$ | 12 | 5 / 5 | 5 / 10 | 13 / 17 |
| S AN | 18 | 11 / 10 | 10 / 19 | 22 / 26 |
| S AN+$C_3H_6$ | 51 | 77 / 79 | 72 / 61 | 52 / 54 |
| S COX | 0 | 0 / 0 | 0 / 4 | 0 / 0 |

(*) : du mélange gazeux

## Revendications

1. Procédé d'ammoxydation d'alcanes en phase vapeur en présence d'un catalyseur solide, caractérisé en ce que la phase active dudit catalyseur répond à la formule empirique $Mo_aV_bM_cO_x$ dans laquelle :
   - M représente un ou plusieurs éléments choisis parmi le manganèse, le zinc, le cobalt, le cuivre, le lithium, le sodium, le potassium et l'argent.
   - a est un nombre non nul et inférieur à 2 ;
   - b est un nombre non nul et inférieur à 2 ;

12

- c est un nombre non nul et inférieur à 1 ; et
- x est déterminé par le degré d'oxydation des autres éléments.

2. Procédé selon la revendication 1, caractérisé en ce que ladite phase active répond à ladite formule empirique dans laquelle M représente un ou plusieurs éléments dont au moins un est choisi parmi le manganèse, le zinc et le cobalt.

3. Procédé selon la revendication 1 ou 2, caractérisé en ce que ladite phase active répond à la formule empirique dans laquelle :
   - a est un nombre compris entre 0,1 et 1 inclus ;
   - b est un nombre compris entre 0,05 et 1,8 inclus ; et
   - c est un nombre compris entre 0,1 et 1 inclus.

4. Procédé selon l'une quelconque des revendications 1 à 3 caractérisé en ce que ladite phase active répond à la formule empirique dans laquelle :
   - M représente un élément choisi parmi le manganèse, le zinc et le cobalt
   - a est un nombre compris entre 0,1 et 1 inclus ;
   - b est un nombre compris entre 0,05 et 1,8 inclus ; et
   - c est un nombre compris entre 0,1 et 1 inclus.

5. Procédé selon l'une quelconque des revendications précédentes, caractérisé en ce que l'alcane est le propane.

6. Procédé selon l'une quelconque des revendications précédentes, caractérisé en ce que la réaction est conduite en présence de vapeur d'eau.

7. Procédé selon l'une quelconque des revendications précédentes, caractérisé en ce que la température de réaction est comprise entre 350°C et 550°C.

8. Procédé selon l'une quelconque des revendications précédentes, caractérisé en ce que la pression totale est comprise entre 1 et 6 Bar.

9. Procédé selon l'une quelconque des revendications précédentes, caractérisé en ce que la vitesse volumique horaire est comprise entre 100 et 36 000 $h^{-1}$.

10. Procédé selon l'une quelconque des revendications précédentes, caractérisé en ce que dans le gaz réactif (mélange d'hydrocarbure saturé, d'ammoniac et d'oxygène) la teneur en hydrocarbure saturé est comprise entre 5 et 70 %, la teneur en ammoniac est comprise entre 3 et 45 % et la teneur en oxygène est comprise entre 3 et 45 %.

11. Procédé selon l'une quelconque des revendications précédentes, caractérisé en ce que la composition du mélange gazeux est en dehors du domaine d'explosivité.

12. Procédé selon l'une quelconque des revendications précédentes, caractérisé en ce que le catalyseur solide renferme, outre ladite phase active, un support.

**Claims**

1. Process for the ammoxidation of alkanes in the vapour phase in the presence of a solid catalyst, characterized in that the active phase of the said catalyst corresponds to the empirical formula $Mo_aV_bM_cO_x$, in which:
   - M represents one or a number of elements chosen from manganese, zinc, cobalt, copper, lithium, sodium, potassium and silver;
   - a is a non-zero number less than 2;
   - b is a non-zero number less than 2;
   - c is a non-zero number less than 1; and
   - x is determined by the oxidation number of the other elements.

EP 0 547 981 B1

2. Process according to claim 1, characterized in that the said active phase corresponds to the said empirical formula in which M represents one or a number of elements of which at least one is chosen from manganese, zinc and cobalt.

3. Process according to claim 1 or 2, characterized in that the said active phase corresponds to the empirical formula in which:
   - a is a number between 0.1 and 1 inclusive;
   - b is a number between 0.05 and 1.8 inclusive; and
   - c is a number between 0.1 and 1 inclusive.

4. Process according to any one of claims 1 to 3, characterized in that the said active phase corresponds to the empirical formula in which:
   - M represents an element chosen from manganese, zinc and cobalt;
   - a is a number between 0.1 and 1 inclusive;
   - b is a number between 0.05 and 1.8 inclusive; and
   - c is a number between 0.1 and 1 inclusive.

5. Process according to any one of the preceding claims, characterized in that the alkane is propane.

6. Process according to any one of the preceding claims, characterized in that the reaction is carried out in the presence of steam.

7. Process according to any one of the preceding claims, characterized in that the reaction temperature is between 350°C and 550°C.

8. Process according to any one of the preceding claims, characterized in that the total pressure is between 1 and 6 bar.

9. Process according to any one of the preceding claims, characterized in that the hourly volume rate is between 100 and 36,000 $h^{-1}$.

10. Process according to any one of the preceding claims, characterized in that, in the reactive gas (mixture of saturated hydrocarbon, ammonia and oxygen), the content of saturated hydrocarbon is between 5 and 70 %, the content of ammonia is between 3 and 45 % and the content of oxygen is between 3 and 45 %.

11. Process according to any one of the preceding claims, characterized in that the composition of the gaseous mixture is outside the explosive region.

12. Process according to any one of the preceding claims, characterized in that the solid catalyst contains, besides the said active phase, a support.

**Patentansprüche**

1. Verfahren zur Ammoxidation von Alkanen in der Dampfphase und in Anwesenheit eines festen Katalysators, dadurch gekennzeichnet, daß die aktive Phase des genannten Katalysators der empirischen Formel $Mo_aV_bM_cO_x$ entspricht, in der
   - M ein oder mehrere Elemente darstellt, ausgewählt unter Mangan, Zink, Cobalt, Kupfer, Lithium, Natrium, Kalium und Silber,
   - a eine Zahl ungleich null und unter 2 darstellt,
   - b eine Zahl ungleich null und unter 2 darstellt,
   - c eine Zahl ungleich null und unter 1 darstellt, und
   - x durch den Oxidationsgrad der anderen Elemente bestimmt wird.

2. Verfahren nach Anspruch 1, dadurch gekennzeichnet, daß die genannte aktive Phase der genannten empirischen Formel entspricht, in der M ein oder mehrere Elemente darstellt, von denen mindestens eines unter Mangan, Zink und Cobalt ausgewählt wird.

14

3. Verfahren nach Anspruch 1 oder 2, dadurch gekennzeichnet, daß die genannte aktive Phase der empirischen Formel entspricht, in der
   - a eine Zahl zwischen 0,1 und einschließlich 1 darstellt,
   - b eine Zahl zwischen 0,05 und einschließlich 1,8 darstellt, und
   - c eine Zahl zwischen 0,1 und einschließlich 1 darstellt.

4. Verfahren nach irgendeinem der Ansprüche 1 bis 3, dadurch gekennzeichnet, daß die genannte aktive Phase der empirischen Formel entspricht, in der
   - M ein unter Mangan, Zink und Cobalt ausgewähltes Element darstellt,
   - a eine Zahl zwischen 0,1 und einschließlich 1 darstellt,
   - b eine Zahl zwischen 0,05 und einschließlich 1,8 darstellt, und
   - c eine Zahl zwischen 0,1 und einschließlich 1 darstellt.

5. Verfahren nach irgendeinem der vorstehenden Ansprüche, dadurch gekennzeichnet, daß das Alkan Propan ist.

6. Verfahren nach irgendeinem der vorstehenden Ansprüche, dadurch gekennzeichnet, daß die Reaktion in Anwesenheit von Wasserdampf durchgeführt wird.

7. Verfahren nach irgendeinem der vorstehenden Ansprüche, dadurch gekennzeichnet, daß die Reaktionstemperatur zwischen 350 °C und 550 °C liegt.

8. Verfahren nach irgendeinem der vorstehenden Ansprüche, dadurch gekennzeichnet, daß der Gesamtdruck zwischen 1 und 6 bar liegt.

9. Verfahren nach irgendeinem der vorstehenden Ansprüche, dadurch gekennzeichnet, daß die Stundenvolumen-Geschwindigkeit zwischen 100 und 36.000 $h^{-1}$ liegt.

10. Verfahren nach irgendeinem der vorstehenden Ansprüche, dadurch gekennzeichnet, daß in dem reaktiven Gas (Mischung von gesättigtem Kohlenwasserstoff, Ammoniak und Sauerstoff) der Gehalt an gesättigtem Kohlenwasserstoff zwischen 5 und 70 %, der Gehalt an Ammoniak zwischen 3 und 45 % und der Gehalt an Sauerstoff zwischen 3 und 45 % liegt.

11. Verfahren nach irgendeinem der vorstehenden Ansprüche, dadurch gekennzeichnet, daß die Zusammensetzung der Gasmischung außerhalb des Bereiches der Explosionsfähigkeit liegt.

12. Verfahren nach irgendeinem der vorstehenden Ansprüche, dadurch gekennzeichnet, daß der feste Katalysator außer der genannten aktiven Phase einen Träger umfaßt.

FIGURE 1

FIGURE 2